Europäisches Patentamt

European Patent Office

Office européen des brevets

(19)

(11) Publication number: **0 192 626 B1**

# EUROPEAN PATENT SPECIFICATION

(45) Date of publication of patent specification: **29.07.92**

(21) Application number: **86870014.7**

(22) Date of filing: **03.02.86**

(51) Int. Cl.5: **C12N 15/30**, C12N 15/62,
C12N 1/21, C12P 21/02,
//(C12N1/21,C12R1:19)

(54) **Malaria vaccine.**

(30) Priority: **07.02.85 US 699116**

(43) Date of publication of application:
**27.08.86 Bulletin 86/35**

(45) Publication of the grant of the patent:
**29.07.92 Bulletin 92/31**

(84) Designated Contracting States:
**AT BE CH DE FR GB IT LI LU NL SE**

(56) References cited:
EP-A- 0 153 188      EP-A- 0 166 410
WO-A-84/02917        WO-A-84/02922
WO-A-86/00911        US-A- 4 466 917

SCIENCE, vol. 225, 10th August 1984, pages
593-599; J.B. DAME et al.: "Structure of the
gene encoding the immunodominant sur-
face antigen on the sporozoite of the human
malaria parasite Plasmodium falciparum"

(73) Proprietor: **SMITHKLINE BEECHAM CORPORA-
TION**
**P.O. Box 7929 1 Franklin Plaza**
**Philadelphia Pennsylvania 19101(US)**

Proprietor: **The United States of America as
respresented by the Secretary of the Army
One Franklin Plaza
Philadelphia, Pennsylvania 19103(US)**

(72) Inventor: **Ballou, Ripley W.**
**1909 Stratton Road
Silver Spring Maryland 20910(US)**
Inventor: **Gross, Mitchell Stuart**
**667 Pugh Road
Wayne Pennsylvania 19087(US)**
Inventor: **Hockmeyer, Wayne T.**
**8602 Garfield Street
Bethesda Maryland 20817(US)**
Inventor: **Young, James Francis**
**10 Holbrook Road
Havertown Pennsylvania 19083(US)**

SCIENCE, vol. 225, 10th August 1984, pages 628-629; V. ENEA et al.: "DNA cloning of Plasmodium falciparum circumsporozoite gene: Amino acid sequence of repetitive epitope"

SCIENCE, vol. 228, 24th May 1985, pages 958-962; J.F. YQUNG et al.: "Expression of Plasmodium falciparum circumsporozoite proteins in Escherichia coli for potential use in a human malaria vaccine"

(74) Representative: **Dalton, Marcus Jonathan William et al**
**SmithKline Beecham, Corporate Patents,**
**Great Burgh, Yew Tree Bottom Road**
**Epsom, Surrey KT18 5XQ(GB)**

2

**Description**

BACKGROUND OF THE INVENTION

Malaria is a severe, widespread disease for which, despite years of extensive efforts, a vaccine has not been developed. See, for example, Science, Volume 226, page 679 (November 9, 1984). Experimentally, mammals, including man, have been protected against infection by the etiologic agent of malaria, Plasmodium, by vaccination with irradiated sporozoites. Clyde et al., Am. J. Trop. Med. Hyg., Volume 24, page 397 (1975) and Rieckman et al., Bull, WHO, Volume 57 (Supp. 1), page 261 (1979). Yoshida et al., Science, Volume 207, page 71 (1980) report that such protection is at least partially mediated by antibody directed against a protein on the surface of the sporozoite, the circumsporozoite (CS) protein; monoclonal antibodies raised against CS proteins neutralize infectivity in vitro and protect animals in vivo.

The CS protein appears to be highly evolutionarily conserved within species, but is quite varied across species.

Four species of Plasmodium are known to infect man. These are P. falciparum, P. vivax, P. ovale and P. malariae, the latter two occurring at much lower frequency. Other species of scientific interest are P. berghei and P. knowlesi, the hosts of these species being, respectively, rodents and monkeys.

The CS protein of P. knowlesi comprises twelve tandem repeats of a twelve amino acid sequence. Zavala et al., J. Exp. Med., Volume 157, page 1947 (1983), report that the repeat unit is the major immunogen on the P. knowlesi CS protein, based on experiments showing that monoclonal antibodies to the repeat unit blocked access of anti-sporozoite antisera to solubilized sporozoite protein. Gysin et al, J. Exp. Med., Volume 160, page 935 (1984), reported that a synthetic 24 residue peptide representing tandem repeat units of the P. knowlesi CS protein neutralized infectivity of virulent sporozoites in monkeys.

Colman et al., WO 84-2922-A, published August 2, 1984, report cloning of a portion of the coding region for the P. knowlesi CS protein repeat unit and expression of beta-lactamase and beta-galactosidase fusions thereof in E. coli. Nussenzweig et al., U.S. 4,466,917, disclose a sporozoite protein referred to as the P44 protein and its cloning and expression in E. coli.

Enea et al., Proc. Natl. Acad. Sci. USA, Volume 81, page 7520 (1984), report an analogous repeat unit structure within the CS protein of P. cynomologi.

Kemp et al., WO.84-02917-A, disclose cloning and expression of P. falciparum cDNA in E. coli.

Dame et al., Science, Volume 225, page 593 (1984), report cloning and expression of the CS protein or P. falciparum in E. coli. The protein is described as comprising about 412 amino acids with an approximate molecular weight of 44,000. It comprises 41 tandem repeats of a tetrapeptide. Synthetic 7-, 11- and 15-residue peptides derived from the repeat region bound to monoclonal antibodies raised against the CS protein.

SUMMARY OF THE INVENTION

In one aspect, the invention is an immunogenic polypeptide capable of conferring immunity in mammals to infection by Plasmodium falciparum comprising four or more tandem repeat units of a Plasmodium falciparum CS protein.

In another aspect, the invention is a vaccine for protecting humans against infection by Plasmodium falciparum sporozoites comprising an immunoprotective amount of the polypeptide of the invention and a pharmaceutically acceptable carrier.

BRIEF DESCRIPTION OF THE FIGURE

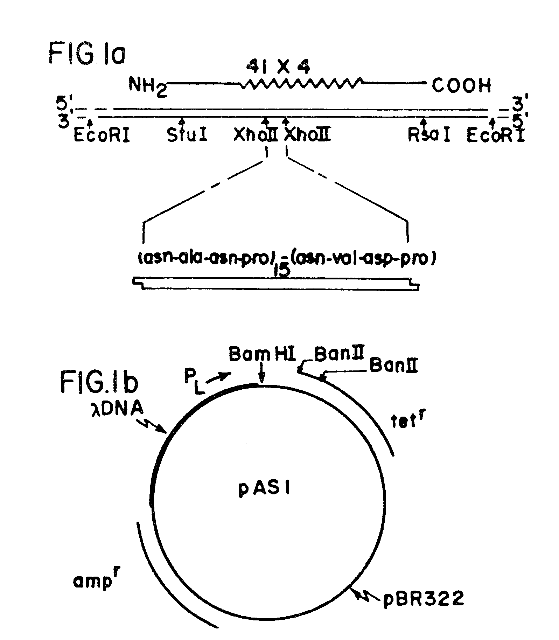

Figure 1a is a partial restriction endonuclease cleavage map of a region of P. falciparum genomic DNA which carries the coding sequence for the CS protein.

Figure 1b is a partial restriction endonuclease cleavage map of pAS1.

DETAILED DESCRIPTION OF THE INVENTION

The polypeptide of this invention comprises tandem CS protein repeat units produced in E. coli. fused to a non- CS protein repeat sequence. The P. falciparum repeat unit is a tetrapeptide having the following sequence:

asparagine(asn)-alanine(ala)-asn-proline(pro)-.

As disclosed by Dame et al., Science, Volume 225, page 593 (1984), of the 41 tetrapeptide repeats in the naturally occurring P. falciparum CS protein, 37 are asn-ala-asn-pro and 4 are asn-valine (val)-aspartic acid (asp)-pro.

The polypeptide of the invention comprises

$[(\text{Asa-Ala-Asn-Pro})_{15}(\text{Asn-Val-Asp-Pro}_1)]_n$ wherein $n \geq 1$.

It preferably comprises from about 16 to about 112 repeats ($_{15}n = 1\text{-}7$).

The polypeptide of the invention a hybrid, that is, a fusion polypeptide, having non-CS protein repeat unit sequences. Such non-CS protein repeat sequence serves as a carrier molecule to enhance immunogenicity or to facilitate cloning and expression in recombinant microorganisms.

Specific embodiments of types of polypeptides of the invention exemplified herein are:

$\text{Rtet}_{32}$ polypeptides, which comprise at least 4 repeats with about 32 N-terminal amino acids from the tetracycline resistance (tetR) gene in pBR322 fused to the C-terminus of the repeats;

$\text{Rtet}_{86}$ polypeptides, which comprise at least 4 repeats with about 86N-terminal amino acids from the tetracycline resistance tetR gene in pBR322 fused to the C-terminus of the repeats;

RNS1 polypeptides which comprise at least 4 repeats with the 227 amino acids of NS1 (influenza virus nonstructural protein 1) fused to the C-terminus of the repeats;

NS1R polypeptides, which comprise at least 4 repeats with 81 N-terminal amino acids of NS1 fused to the N-terminus of the repeats;

RG polypeptides which comprise at least 4 repeats followed by a -glycine residue at the C-terminus of the repeats;

RLA polypeptides which comprise at least 4 repeats followed by -leucine-arginine residues at the C-terminus of the repeats; and

RN polypeptides, which comprise at least 4 repeats followed by -asn-thr-val-ser-ser at the C-terminus of the repeats.

A genetic coding sequence for the CS protein repeat units can be obtained by known techniques. These include synthesis and, preferably, by obtainment from P. falciparum by reverse transcription of messenger RNA as disclosed, for example, by Ellis et al., Nature, Volume 302, page 536 (1983), or by directly cloning the intact gene from P. falciparum genomic DNA as disclosed, for example, by Dame et al., cited previously. The Figure illustrates the CS protein coding region. P. falciparum, and sporozoites thereof, can be obtained from infected humans and mosquitoes.

Having cloned the coding sequence for all or part of the CS protein, a sub-fragment thereof coding for all or a portion of the repeat unit can be prepared by known techniques. Figure 1a shows selected available restriction sites within the CS protein gene. Preferred sites are the Xho II sites. Cutting with Xho II releases a coding sequence for 16 repeats as follows:

$\text{N-asp-pro}[(\text{asn-ala-asn-pro})_{15}(\text{asn-val-asp-pro})_1]_n\text{C}$.

wherein n is one. Use of multiple tandem Xho II fragments in proper orientation results in longer repeats, that is, n is greater than one.

Techniques for synthesizing are well-known and can be accomplished using commercially available DNA synthesizers. A synthetic oligonucleotide, having codons for substantially the same amino acids and having the same Xho II ends or different cleavage sites at the ends, can be synthesized. Such synthetic oligonucleotides may vary from the natural 64 codons and may code for the same amino acids or for a polypeptide having a small number, preferably less than about 8, different amino acids, provided these do not significantly adversely affect the immunoprotectiveness of the polypeptide. An exemplary synthetic coding sequence codes entirely for the consensus sequence, $(\text{asn-ala-asn-pro-})_n$, wherein n is at least 4.

The coding sequence for the polypeptide can be inserted into any E. coli expression vector, many of which are known and available. The high level of expression of the polypeptides of the invention in E. coli is surprising in view of the unusual amino acid composition of the products - about 50% asparagine (asn), 25% alanine (ala) and 25% proline (pro). As described further below, it has been found that the coding sequence is expressed well using a regulatory element comprising the PL promoter of lambda and the cII ribosome binding site of lambda, as comprised by the plasmid pAS1, described by Rosenberg et al., Meth. Enzym., Volume 101, page 123 (1983) and Shatzman et al., in Experimental Manipulation of Gene Expression, edit. by M. Inouye, Academic Press, New York, 1982. pAS1 carries the pBR322 origin of replication, an ampicillin resistance marker and a series of fragments from lambda, including PL, N antitermination function recognition sites (NutL and NutR), the rho-dependent transcription termination signal (tR1) and the cII ribosome binding site, including the cII translation initiation site, the G residue of which is followed immediately by a Bam HI cleavage site. pAS1 can be derived from pKC30cII by deleting nucleotides between the Bam HI site at the cII-pBR322 junction of pKC30cII and the cII ATG and religating the molecule to regenerate the Bam HI site immediately downstream of the ATG. pKC30cII is constructed by inserting a 1.3 kb Hae III fragment from lambda which carries the cII gene into the Hpa I site of pKC30. See Shatzman et al., cited above, and Rosenberg et al., cited above. pKC30 is described by Shimitake et al., Nature, Volume 292, page 128 (1981). It is a pBR322 derivative having a 2.4 kb Hind III-Bam HI fragment of lambda inserted between the Hind III and Bam HI sites in the tetR gene of pBR322. A construction similar to pAS1 is described by Courtney et al., Nature, Volume 313, page 149 (1985). The coding sequence is operatively linked, that is, in correct orientation and in proper reading frame, to a regulatory element of an E. coli expression vector by standard techniques to construct an expression vector of the invention.

The polypeptide so expressed is isolated and purified from the producing culture by standard protein isolation techniques, many of which are well known in the art. An exemplary, useful purification scheme comprises 1) disruption of cells, 2) clarification of cellular debris, 3) separation of the polypeptides of the invention from other polypeptides present in the clarified cell extract and 4) final purification to remove residual contaminants including residual polypeptides, carbohydrates, nucleic acids and/or lipopolysaccharides.

The first step can be accomplished such as by addition of lysozyme or other lysing or permeabilizing agent or by mechanical or ultrasonic disruption. Prior to centrifugation or filtration to clarify the extract, a surfactant is added to keep the polypeptide of the invention in solution.

As one aspect of the present invention, it has been discovered that certain of the polypeptides of the invention can very efficiently be separated from other polypeptides by heating the clarified extract to about 80°C following addition of a detergent to maintain solubility of the protein. Heating to 80°C for at least about 4 minutes was discovered to cause nearly all bacterial polypeptides to precipitate without denaturing polypeptides comprised substantially of the repeats or of the repeats fused to other non-heat-denaturable sequences. The denatured bacterial polypeptides can be pelleted by centrifugation and removed. This procedure has been used to purify $Rtet_{32}$, RG, RLA and $Rtet_{86}$ polypeptides. In particular, this procedure was used to purify successfully $R16tet_{32}$, $R32tet_{32}$, $R48tet_{32}$, $R64tet_{32}$, R48G, R32LA and $R16tet_{86}$, as described in the Examples, below, but heating of R16NS1 and R32NS1 resulted in precipitation of these polypeptides.

The polypeptide of the invention can be further purified such as by addition of a selective precipitating agent, followed by a final chromatographic step such as ion exchange chromatography or reverse phase HPLC.

In the vaccine of the invention, an aqueous solution of the polypeptide of the invention, preferably buffered at physiological pH, can be used directly. Alternatively, the polypeptide, with or without prior lyophilization, can be admixed or adsorbed with any of the various known adjuvants. Such adjuvants include, among others, aluminum hydroxide, muramyl dipeptide and saponins such as Quil A. As a further exemplary alternative, the polypeptide can be encapsulated within microparticles such as liposomes. In yet another exemplary alternative, the polypeptide can be conjugated to an immunostimulating macromolecule, such as killed Bordetella or a tetanus toxoid.

Vaccine preparation is generally described in New Trends and Developments in Vaccines, edited by Voller et al., University Park Press, Baltimore, Maryland, U.S.A., 1978. Encapsulation within liposomes is described, for example, by Fullerton, U.S. Patent 4,235,877. Conjugation of proteins to macromolecules is disclosed, for example, by Likhite, U.S. Patent 4,372,945 and by Armor et al., U.S. Patent 4,474,757. Use of Quil A is disclosed, for example, by Dalsgaard et al., Acta. Vet. Scand., Volume 18, page 349 (1977).

The amount of polypeptide present in each vaccine dose is selected as an amount which induces an immunoprotective response without significant, adverse side effects in typical vaccinees. Such amount will vary depending upon which specific polypeptide is employed and whether or not the vaccine is adjuvanted. Generally, it is expected that each dose will comprise 1 - 1000 ug of polypeptide, preferably 10 - 200 ug. An optimal amount for a particular vaccine can be ascertained by standard studies involving observation of antibody titres and other responses in subjects. Following an initial vaccination, subjects will preferably receive a boost in about 4 weeks, followed by repeated boosts every six months for as long as a risk of infection exists.

The following Examples are illustrative, and not limiting, of the invention. The CS protein coding sequence was supplied by James Weber, Walter Reed Army Institute for Research, as a 2337 bp Eco RI fragment (see, Fig. 1a) of 2mPF1 (Dame et al., cited above) in the Eco RI site of pUC8, a standard E. coli cloning vector (available, for example, from Bethesda Research Laboratories, Inc., Gaithersburg, MD). The resulting pUC8 derivative is referred to as pUC8 clone 1.

EXAMPLES

Example 1. CS Protein Derivative

Purified pUC8 clone 1 plasmid DNA (40 ug) was digested with restriction endonucleases StuI and RsaI (100 units of each enzyme) in 400 ul of medium buffer (50 mM Tr is, pH7.5, 50mM NaCl, 1mM dithiothreitol (DTT), 10mM $MgCl_2$) for 1.5 hours at 37°C. The resulting 1216 base pair fragment, encoding all but the first 18 amino acids of the CS protein was isolated by electrophoresis on a 5% polyacrylamide gel (PAGE). Expression vector pAS1 (10 ug) was digested with restriction endonuclease Bam HI (25 units) in 200 ul medium buffer for 1.5 hours at 37°C. The cut plasmid was then treated with DNA polymerase large fragment (Klenow, 5 units; 20 mM Tris-HCl, pH7.5, 7mM $MgCl_2$, 60mM NaCl, 6mM 2-mercaptoethanol and 0.25mM of each of the four deoxynucleotide triphosphates; 25°C, 15 minutes) to end fill the Bam HI site. The CS gene fragment (1 ug) was then ligated into this vector (100ng) in 30 ul ligase buffer (50mM Tris, pH7.5, 1mM DTT, 10mM $MgCl_2$, 100 uM rATP) with one unit of T4-DNA ligase for 16 hours at 4°C. The ligation mixture was transformed into E. coli strain MM294Cl+, and ampicillin resistant colonies were obtained, and screened for insertion of the CS gene fragment into the pAS1. A plasmid with the correct construction (pCSP) was identified and was transformed into E. coli strain N5151 (clts857) and tested for expression of the full length CS protein. (The 18 amino acid deletion at the amino terminus of the protein would correspond to a cleaved signal peptide of the authentic CS protein.) Cells were grown in Luria-Bertani Broth (LB) at 32°C to an absorbance at 650nm ($A_{650}$) of 0.6 and temperature induced at 42°C for 2 hours to turn on transcription of the PL promoter of the expression plasmid and subsequent translation of the CS protein derivative. Cells were sampled in 1 ml aliquots, pelleted, resuspended in lysis buffer (10mM Tris-HCl, pH7.8, 25% (vol/vol) glycerol, 2% 2-mercaptoethanol, 2% sodium dodecyl sulfate (SDS), 0.1% bromophenyl blue) and incubated in a 105°C heating block for 5 minutes. Proteins were separated by SDS-PAGE (13% acrylamide, 30:0.8 acrylamide: bis-acrylamide ratio), Proteins were transferred to nitrocellulose and the CS protein produced in E. coli was detected by western blot analysis using a pool of five monoclonal antibodies reactive with the tetrapeptide repeat domain of the P. falciparum CS protein. (Dame et al., cited previously.)

Example 2. R16tet$_{86}$

Purified pUC8 clone 1 plasmid DNA (100 ug) was digested with restriction endonuclease Xho II (40 units) in 400 ul medium buffer at 37°C for 16 hours. A 192 base pair fragment encoding 16 tetrapeptide repeats of the CS protein was then isolated by PAGE. Expression vector pAS1 was cleaved with restriction endonuclease Bam HI as described in Example 1. The 192 base pair Xho II fragment (1 ug) was ligated into the Bam HI site of pAS1 (100ng) as described in Example 1. The ligation mix was transformed into E. coli strain MM294Cl+. A clone was identified which contained a single 192 base pair Xho II fragment in the correct orientation at the Bam HI site of pAS1 by polyacrylamie gel electrophoresis analysis of a Bam HI-Hind II fragment of the plasmid, the Hind II site being downstream of the tetR gene and the Bam HI site being at the juncture of the cII ATG and the insert in correctly oriented plasmids. This plasmid pR16tet$_{86}$ is illustrated as follows:

```
pBR322          PL          repeat              tetR       S      pBR322

    • • •      _____       • • •


                    .

                  BH                           B  B
```

wherein BH represents a Bam HI site, B represents a Ban II site and S, a termination codon. The pR16tet$_{86}$ was used to transform E. coli strain N5151 (clts857) and examined for production of the CS protein tetrapeptide repeat by western blot analysis. The protein so produced had the following sequence:

N-met-asp-pro(asn-ala-asn-pro)$_{15}$ (asn-val-asp-pro)$_1$ T86-C

wherein T86 was 86 amino acids derived from the tetracycline resistance gene present on pAS1. The N-terminal methionine (met) residue was also derived from the vector, more particularly, from the cII protein initiation codon.

Example 2A. R32tet$_{86}$ and R48tet$_{86}$

Purified pR16tet$_{86}$ plasmid DNA (10 ug) was digested with 25 units of Bam HI in 200 ul of medium buffer for 2 hours at 37°C. One hundred ng of this DNA was then ligated with 1 ug of the 192 base pair Xho II fragment as described above. Plasmid expression vectors, pR32tet$_{86}$ and pR48tet$_{86}$, coding for the following polypeptides were prepared and expressed in E. coli.

N-met-asp-pro[(asn-ala-asn-pro)$_{15}$-(asn-val-asp-pro)$_1$]$_n$-T86-C

wherein n is 2 (R32tet$_{86}$) or n is 3 (R48tet$_{86}$). pAS1 clones wherein n was 2 or 3 were selected from clones in which n was other than 2 or 3, respectively, as described above. All clones examined had the insert in the correct orientation. Both R32tet$_{86}$ and R48tet$_{86}$ were expressed at approximately the same levels as R16tet$_{86}$, as estimated by immunoblotting.

Immunoblot analysis of several of the Rtet$_{86}$ proteins revealed a heterogeneous set of products which could not be seen by Coomassie Brilliant Blue R-250 staining. These proteins appeared to have accumulated to roughly half the amounts of the Rtet$_{32}$ polypeptides, described below. It appeared that the sizes of the smallest degradation products were proportional to the number of tetrapeptide repeats in the clones. The instability of these proteins may be due to degradation of the heterologous COOH-terminal tail.

Example 3. R16tet$_{32}$

Purified pR16tet$_{86}$ DNA (10 ug) was cut with 25 units of restriction endonuclease Ban II in 200 ul of medium buffer for 2 hours at 37°C. One hundred nanograms of the cut DNA was then ligated closed. This manipulation resulted in the deletion of a 14 base pair Bam II fragment and produced a termination codon just downstream of the remaining Ban II site. The resulting plasmid, pR16tet$_{32}$, was used to express R16tet$_{32}$ in E. coli strain N5151 and R16tet$_{32}$ was purified therefrom. Thirty grams (wet weight) of E. coli containing R16tet$_{32}$ were resuspended in 200 ml buffer A (50mM Tris HCl, pH 8.0, 2mM ethylenediaminetetraacetic acid (EDTA), 0,1 mM dithiothreitol, 5% (vol/vol) glycerol). Lysozyme was added to a final concentration of 0.2 mg/ml, and the mixture was incubated on ice for 30 minutes to lyse cells. The mixture was then treated in a Waring blender for 3 minutes at the high setting followed by sonication for one minute with a Branson 350 sonifier to shear bacterial DNA. Sodium deoxycholate was added to a final concentration of 0.1% (w/v), and this mixture was stirred for 30 minutes at 4°C. The suspension was then centrifuged at 12,000 x g for 30 minutes to remove cell debris. The supernatant was collected in a flask, incubated in a boiling water bath for 10 minutes, and centrifuged at 12,000 x g for 30 minutes. It was found that nearly all E. coli proteins precipitated during the heat step and pelleted during the centrifugation, whereas, the R16tet$_{32}$ protein was soluble and was contained in the supernatant. The supernatant was collected and ammonium sulfate was then slowly added to a final concentration of 20% of saturation. This resulted in selective precipitation of the R16tet$_{32}$ protein which was then collected by centrifugation (12,000 x g for 30 minutes). At this point the R16tet$_{32}$ protein was greater than about 95% pure with respect to other contaminating bacterial proteins.

A final chromatographic step (e.g., ion exchange, reverse phase high performance liquid chromatography, phenyl sepharose® chromatography, size separation, etc.) can then be performed to remove residual contamination by other materials such as proteins, carbohydrates, nucleic acids or lipopolysaccharides. R16tet$_{32}$ was expressed and purified at levels approximately equal to 5% of total E. coli protein, that is, about 30-60 mg/L, as shown by Coomasie Blue Staining.

R16tet$_{32}$ has the following sequence:

N-met-asp-pro[(asn-ala-asn-pro)$_{15}$ (asn-val-asp-pro)$_1$]$_n$T32-C

wherein n is one and T32 is 32 amino acids derived from the tetracycline resistance gene. More particularly, T32 has the following sequence:

7

```
-leu-arg-arg-thr-his-arg-gly-arg-his-his-arg-arg-his-arg-cys
-gly-cys-trp-arg-leu-tyr-arg-arg-his-his-arg-trp-gly-arg-ser
-gly-ser-C
```

the remaining Ban II site being between residues 30 and 31.

Example 3A. R32tet$_{32}$, R48tet$_{32}$

Substantially as described in Example 3, above, R32tet$_{32}$ and R48tet$_{32}$, (R16tet$_{32}$ in which n is 2 and 3, respectively), were expressed in E. coli and isolated to the same level and degree of purity as R16tet$_{32}$. The starting vectors were pR32tet$_{86}$ and pR48tet$_{86}$, respectively.

Example 3B. R64tet$_{32}$, R80tet$_{32}$

Purified pR48tet$_{32}$ plasmid DNA (10 ug) was digested with 25 units of Bam HI in 200 ul of medium buffer for 2 hours at 37°C. One hundred nanograms of this DNA was then ligated with 1 ug of the 192 base pair Xho II fragment as described above. Plasmid expression vectors coding for the following polypeptides were prepared and expressed in E. coli.

N-met-asp-pro[(asn-ala-asn-pro)$_{15}$ (asn-val-asp-pro)$_1$ ]$_n$-T32-C

wherein n is 4 (R64tet$_{32}$) or n is 5 (R80tet$_{32}$). pAS1 clones wherein n was 4 or 5 were selected from clones in which n was other than 4 or 5, respectively, as described above. Both R64tet$_{32}$ and R80tet$_{32}$ expressed at approximately the same levels as R48tet$_{32}$. R64tet$_{32}$ was purified in substantially the same manner as R16tet$_{32}$, R32tet$_{32}$ and R48tet$_{32}$, described above.

Example 3C. R96tet$_{32}$ and R112tet$_{32}$

Substantially as described in Example 3B, above, R96tet$_{32}$ and R112tet$_{32}$ (in which n is 6 and 7, respectively), were expressed in E. coli at approximately the same levels as R48tet$_{32}$. The starting vector was pR80tet$_{32}$.

Although some heterogeneity in purified Rtet$_{32}$ polypeptides was observed by immunoblot analysis, the major reactive species correlated with the band seen by protein staining. The observed molecular weights by SDS-PAGE were approximately twice that expected, although the migration of each of the proteins was proportional to the number of tetrapeptide repeat units in each of the constructs. Amino acid composition determinations on several Rtet$_{32}$ polypeptides were consistent with expected values.

Example 4. R16G

pTerm was prepared by inserting a synthetic linker with the following sequence:

```
5'-GATCCCGGGTGACTGACTGA          -3'
3'-       GGCCCACTGACTGACTCTAG  -5'
```

into the Bam HI site of pAS1. pAS1 (10 ug) was digested with 25 units of Bam HI. One hundred ng of the Bam HI-cut pAS1 was ligated with 20 nanograms of the synthetic linker and plasmid pTerm was identified with one linker inserted into the Bam HI site of pAS1. This vector retains the Bam HI site and results in the insertion of TGA termination codons downstream of the ATG initiation codon of the cII protein in all three reading frames.

pR16G was prepared by inserting the 192 base pair Xho II fragment from pUC8 clone 1 into the Bam HI site of pTerm and a clone having a single Xho II insert in the proper orientation was selected substantially as described previously.

pR16G was cloned and expressed in E. coli strain N5151, substantially as described above.

R16G has the following sequence:

N-met-asp-pro[(asn-ala-asn-pro)$_{15}$-(asn-val-asp-pro)$_1$ ]$_n$-gly-C

wherein n is one.

Since this protein does not contain any aromatic residues it cannot be visualized by Coomassie Brilliant Blue R-250 staining to quantitate expression levels. By immunoblot analysis with 5 monoclonal antibodies specific for the CS protein (Dame et al., cited previously), levels were estimated to be approximately 1% of total cell protein as compared to R16tet$_{32}$, with which visualization by Coomassie Brilliant Blue R-250 staining is possible.

Example 4A. R32G, R48G, R64G, R80G and R112G

R32G, R48G, R64G, R80G and R112G (R16G in which n is 2, 3, 4, 5 or 7, respectively) were expressed in E. coli strain N5151 as described in Example 4, above. These polypeptides were expressed at about the same level as R16G. R48G was purified substantially as described in Example 3.

Example 5. R16LA and R32LA

pTerm2 was prepared by inserting a synthetic linker with the following sequence:

$$\text{5'-GATCCGCTGCGTT} \quad \text{-3'}$$
$$\text{3'-} \quad \text{GCGACGCAACTAG-5'}$$

into the Bam HI site of pAS1, substantially as described in Example 4. pTerm2 retains the Bam HI site. The 192 base pair Xho II fragment from pUC8 clone 1 was inserted as described above. pR16LA and pR32LA, clones having one or two Xho II inserts in the proper orientation, respectively, were selected substantially as described previously. R32LA was purified substantially as described in Example 3.

pR16LA and pR32LA were cloned and expressed in E. coli strain N5151, substantially as described previously.

R16LA and R32LA have the following sequence:

N-met-asp-pro[(asn-ala-asn-pro)$_{15}$ (asn-val-asp-pro)$_1$ ]$_n$-leu-arg-C

wherein n is 1 and 2, respectively. The C-terminal leucine and arginine derive from the synthetic linker in pTerm2. The R16LA was expressed as about 1% of total E. coli protein, whereas, R32LA was expressed at approximately 5% of total cell protein.

Example 6. R16NS1

pAS1deltaEH was prepared by deleting a non-essential Eco RI - Hind III region of pBR322 origin from pAS1. Ten micrograms of pAS1 was cut with Eco RI and Hind III (20 units each) in 200 ul of medium buffer, treated with DNA polymerase (Klenow), ligated closed, and transformed into E. coli, substantially as described above. A clone with the 29 base pair Eco RI - Hind III fragment deleted was identified. A 1236 base pair Bam HI fragment of pAPR801 (Young et al., Proc. Natl. Acad. Sci. U.S.A., Volume 80, page 6105 (1983)), containing the influenza virus (A/PR/8/34) NS1 coding region within 861 base pairs of viral origin and 375 base pairs of pBR322 origin, was inserted into the Bam H1 site of pAS1deltaEH. The resulting plasmid, pAS1deltaEH/801, expresses authentic NS1 (230 amino acids). This plasmid retains the Bam HI site between the cII translation start site and the NS1 coding sequence.

PAS1deltaEH/801 (10 ug) was cut with Eco RI (20 units) and Sal I (20 units) in 200 ul of high buffer (50mM Tris-HCl, PH7.5, 1mM DTT, 10mM MgCl$_2$, 100mM NaCl) for 2 hours at 37°C, treated with DNA polymerase large fragment (Klenow), and ligated closed, substantially as described above. A clone having the 650 base pair Eco RI-Sal I region deleted was isolated. This plasmid, pNS1deltaES, expresses authentic NS1.

pR16NS1 was prepared by inserting a 192 base pair, Xho II fragment from pUC8 clone 1 into the Bam HI site in pNS1deltaES and clones having a single Xho II insert in proper orientation were selected substantially as described previously.

pR16NS1 was cloned and expressed in E. coli, and R16NS1 was purified, substantially as described above, omitting the boiling step.

R16NS1 has the following sequence:

N-met-asp-pro[(asn-ala-asn-pro)$_{15}$(asn-val-asp-pro)$_1$]$_n$-N227

where n is one and N227 is 227 amino acids of NS1 origin.

R16NS1 in the R16NS1 preparation was estimated to comprise greater than 80% of protein, without the boil or ion exchange step. R16NS1 represented an especially surprisingly high proportion, approximately 25%, of total cellular protein.

Example 6A. R32NS1, R48NS1 and R64NS1

R32NS1 (R16NS1 in which n is 2) was expressed in and purified from E. coli, substantially as described in Example 3, above, omitting the boiling step. R32NS1 was expressed at about the same level as R16NS1 and purified to about the same degree.

R48NS1 (R16NS1 in which n is 3) and R64NS1 (R16NS1 in which n is 4) were expressed in E. coli substantially as described above. R48NS1 and R64NS1 expressed at about 10% and 5% of total E. coli protein, respectively.

Example 7. NS1R48

pR48tet$_{86}$ was cleaved with Bam HI and end-filled with DNA Polymerase (Klenow) substantially as described above. The plasmid was then cleaved with Ban II as described above, to release a 672 base pair fragment carrying 3 Xho II fragments and 96 base pairs from the tetracycline resistance gene.

Ten micrograms of pAS1deltaEH/801 was cut with Nco I (20 units) in 200 ul of high buffer for 2 hours at 37°C, and end-filled with DNA polymerase large fragment (Klenow) substantially as described above. The Nco I site is in the codon for residue 81 in NS1. The plasmid was then cut with Ban II, as described above to delete the remaining NS1 codons and a portion of the tetracycline resistance gene, to produce pAS1deltaEH/801-1.

The 672 base pair, Bam HI (end-filled)-Ban II fragment was inserted into pAS1deltaEH/801-1 to prepare pNS1R48. This plasmid was expressed in E. coli, substantially as described above. NS1R48 has the following sequence:

N-81N-asp-pro[(asn-ala-asn-pro)$_{15}$(asn-val-asp-pro$_1$]$_n$ T32-C

wherein 81N is 81 N-terminal amino acids of NS1, n is 3 and T32 is as described above. NS1R48 was expressed as about 5% of total cellular protein.

Example 8. R32N

Ten micrograms of pR32NS1 was cut with Hind III (25 units) in 200 ul of medium buffer for 2 hours at 37°C, and end-filled with DNA polymerase substantially as described above, to produce pR32NS1-1. The Hind III site is in the codon for residue 5 in the NS1 coding region. pR32NS1-1(100ng) was then ligated closed substantially as described above. The resulting plasmid, pR32N, now contained a TAA termination codon after the fifth codon in the NS1 coding sequence. pR32N was used to express R32N in E. coli substantially as described previously.

R32N has the following sequence:

N-met-asp-pro[(asn-ala-asn-pro)$_{15}$-(asn-val-asp-pro)$_1$]$_n$-N5-C

wherein n is 2 and N5 is 5 amino acids derived from the NS1 gene. More particularly, N5 has the following sequence:

-asn-thr-val-ser-ser-C.

R32N was expressed as about 5% of total E. coli protein.

Example 9. Antibody Response - ELISA

Recombinant proteins R16tet$_{32}$, R32tet$_{32}$ and R48tet$_{32}$ were purified substantially as described above, dialyzed against .01 M phosphate buffered saline, pH 7.0 (PBS), aliquoted, and stored at -80°C. Constructs were mixed with either PBS, aluminum hydroxide (alum) or Complete Freund's Adjuvant (CFA) to yield a 0.5 ml dose containing 50 ug protein. CFA (GIBCO, Grand Island, New York) plus antigen in PBS were emulsified in a 1:1 ratio by agitation for 30 minutes on a mechanical vortexer. Alum was prepared from aluminum hydroxide gel, USP, diluted in PBS. Antigen was absorbed to alum at 4°C for 12 hours on a rotary mixer. The suspension was allowed to settle for an additional 12 hours and sufficient supernatant was discarded to yield 0.80 mg Al and 50 ug recombinant protein per dose. Six to eight week old C57B1/6 mice were immunized with a total of 50 ug of protein subcutaneously and intraperitoneally (5 animals per group). Animals were boosted 4 weeks after the primary immunizaton following the same protocol as for the first injections, except that the group which had received the immunogens in CFA were boosted with proteins emulsified in Incomplete Freund's adjuvant (IFA). One week later, whole blood obtained by tail bleeding was pooled, clotted overnight at 4°C, and centrifuged to separate the serum. These sera were stored at -80°C until needed.

An enzyme linked immunosorbent assay (ELISA) was used to test all sera for their ability to react with a 16 amino acid synthetic peptide consisting of four repeats of the P. falciparum CS protein (asn-ala-asn-pro)$_4$ Dame et al. (Science, Volume 225, page 593 (1984). Synthetic peptide antigen was coupled to bovine serum albumin (BSA) was used to coat the wells of microtiter plates. Fifty ul (0.1 ug) of the screening antigen diluted with 0.01 M phosphate buffered saline, pH 7.4, (PBS), were pipetted into wells of polystyrene microtitration plates (Immunlon 2 Dynatech Laboratories, Alexandria, VA) and held overnight at room temperature (about 22°C) (RT). Well contents were then aspirated, filled with blocking buffer (BB = 1.0% BSA, 0.5% casein, 0.005% thimersol and 0.0005% phenol red in PBS) and held for 1 hour at RT. Mouse sera were diluted serially in BB and 50 ul was added to each well. After a 2 hour incubation at RT, wells were aspirated, washed three times with PBS-0.05% Tween® 20 (PBS-TW20) and 50 ul of horseradish peroxidase conjugated to goat anti-mouse IgG (H + L) (Bio-Rad Laboratories, Richmond, CA) diluted 1/500 with 10% heat inactivated human serum in PBS was added to each well. After 1 hour, well contents were aspirated, washed three times with PBS-TW20 and 150 ul of substrate (1 mg 2,2'-azino-di(3-ethyl-benzthiazoline sulfonic acid-6) per ml of 0.1 M citrate-phosphate buffer, pH 4.0, with 0.005% hydrogen peroxide added immediately before use) was then added to each well. Absorbance at 414 nm was determined 1 hour later with a ELISA plate reader (Titertek Multiskan, Flow laboratories, Inc., McLean, VA). The R16tet$_{32}$, R32tet$_{32}$ and R48tet$_{32}$ constructs all resulted in the production of antibody which reacted in the ELISA.R16tet$_{32}$, when administered alone, was poorly immunogenic when compared to R32tet$_{32}$ and R48tet$_{32}$. Both alum and CFA enhanced immunogenicity of all three proteins and antibody was detected at titers out to 102,000 in at least one regimen.

Example 10. Antibody Response - IFA

The antisera from Example 9 were shown to react strongly with authentic P. falciparum CS protein which tested in an indirect immunofluorescent antibody assay (IFA). Reactivity against P. knowlesi, P. cynomologi, P. viva, and P. gallinaceum was not detected. A slight reactivity of the antisera to R32tet$_{32}$ was seen with P. berghei. This observation is consistent with previous data by Hockmeyer et al., in Proc. 3d Int'l. Symp. Immunobiol. Proteins Peptides, ed. by Atassi, M.Z., Plenum New York (in press) showing that some Mabs to P. falciparum react with P. berghei sporozoites by IFA.

Sporozoites were dissected from the salivary glands of infected mosquitoes substantially as described by Bosworth, J. Parasitol., Volume 61, page 769 (1975), diluted in saline or Medium 199 (GIBCO)containing 0.5% BSA, counted using a haemacytometer and diluted to 2,000-5,000 sporozoites per 10 ul. Ten ul aliquots were spread onto each well of multi-well printed IFA slides, air dried at room temperature and stored at -80°C.

IFA's were initiated by spreading 20 ul volumes of serum, diluted 1/100 with BB, onto the well of an IFA slide containing dried sporozoites. After a 20 minute incubation in a moist chamber at RT, the serum solutions were aspirated and the spots were washed with 2 drops of PBS. Twenty ul aliquots of goat anti-mouse antibody conjugated to fluorescein isothiocyanate (Kirkegard and Perry, Gaithersburg, MD) diluted 1:40 with BB were then added to each spot. After a second 20 minute incubation at RT the spots were again washed with 2 drops of PBS, mounted in glycerol and examined under UV light at 500X magnification for fluorescence.

Example 11. Circumsporozoite protein (CSP) Reaction

Sera from mice immunized with R16tet$_{32}$, R32tet$_{32}$ and R48tet$_{32}$ produced strong CSP positive reactions (Table 1). When administered without adjuvant, only R16tet$_{32}$ failed to produce antibody which gave positive CSP reactions, whereas, when given with CFA or alum, all three constructs induced antibodies, which produced strong CSP reactions.

Table 1

| CSP Reactivity of Antisera to R16tet$_{32}$, R32tet$_{32}$, and R48tet$_{32}$ | | | |
|---|---|---|---|
| ADJUVANT | Antisera | | |
| | R16tet$_{32}$ | R32tet$_{32}$ | R48tet$_{32}$ |
| NONE | 0/25(-) | 17/25(2 +) | 21/25(4 +) |
| CFA | 23/25(4 +) | 21/25(4 +) | 21/25(4 +) |
| ALUM | 25/25(4 +) | 25/25/(4 +) | 16/27(2 + -4 +) |

CSP reactions were performed essentially as described by Vanderberg et al. Mil. Med. Volume 134 (Supp. 1), page 1183 (1969). Five microliters containing 500-1,000 P. falciparum mosquito salivary gland sporozoites resuspended in Medium 199 were mixed with 5 ul of serum on a microscope slide, sealed under a cover slip rimmed with petroleum jelly and incubated at 37°C. for 1 hour. Reactions were evaluated by phase contrast microscopy at 400X magnification. Twenty-five random sporozoites were examined for each serum sample and the number of CSP positive organisms are indicated. The degree of CSP reactivity as described by Vanderberg et al., cited above, is shown in parentheses. A (-) indicates no CSP reactivity detectable; (2 +) indicates appearance of a granular precipitate on the surface of the sporozoites; (4 +) indicates appearance of a long, thread-like filament at one end of the sporozoites. Normal mouse serum, and serum from mice immunized with CFA alone, produced no detectable CSP reactivity in parallel assays.

Example 12. Hepatocyte Blocking

The sera from Example 9, above, were examined in an in vitro inhibition of invasion assay (Table 2). These data show that the R32tet$_{32}$ and R48tet$_{32}$ proteins induce antibodies with strong blocking activity even in the absence of adjuvant. R16tet$_{32}$ was less efficient in eliciting strong blocking antibodies except when administered adsorbed to alum. This finding is consistent with the poor CSP reactivity and low ELISA titers observed with the antisera raised to the R16tet$_{32}$ protein.

Table 2

| Inhibition of P. falciparum Sporozoite Invasion HepG2 - A16 Hepatoma cells in vitro. | | | |
|---|---|---|---|
| ADJUVANT | Antisera | | |
| | R16 | R32 | R48 |
| NONE | 46 | 95 | 92 |
| CFA | 76 | 92 | 94 |
| ALUM | 100 | 100 | 96 |

Inhibition of sporozoite invasion of cultured cells was performed substantially as previously described by Hollingdale et al. J. Immunol. Volume 32, page 909 (1984). The sera obtained from mice immunized with the R16tet$_{32}$, R32tet$_{32}$ and R48tet$_{32}$ constructs were tested for their ability to inhibit invasion of cultured cells by P. falciparum sporozoites. The sera were diluted in culture medium and added to HepG2-A16 cell cultures to yield a final dilution of 1:20. (V/V). Cultures then received 12,000 to 40,000 mosquito salivary gland P. falciparum sporozoites and were incubated at 37°C in 5% CO$_2$ atmosphere for 3 hours, rinsed wtih Dulbecco's phosphate-buffered saline (PBS), fixed in methanol, and rinsed 2 times with PBS.

Sporozoites that had entered cells were visualized by an immunoperoxidase antibody assay (IPA) (Hollingdale et al., cited above). The IPA was carried out by first treating the fixed cultures with a Mab to P.

12

falciparum (2F1.1, See, Dame et al., cited above) followed by incubation with rabbit anti-mouse immunoglobulin conjugated with horseraddish peroxidase and staining with 3,3-diaminobenzidine. The number of sporozoites that invaded cultured cells was determined by counting the intracellular parasites present in the entire preparation on a Leitz microscope at 250X with a dark blue filter. Experiments were carried out either in duplicate or triplicate and each cell culture within an experiment received an equal number of sporozoites. Inhibition was the percentage reduction of sporozoite invasion by anti-construct immune sera compared to normal mouse serum controls where CS reactive Mab 2F1.1 gave 100% inhibition of sporozoite invasion at dilutions of 1/20.

Recombinant proteins RLA, R16NS1 and R32NS1, prepared substantially as described above, were similarly tested by the ELISA and IFA assays and were shown similarly to induce antibody which reacted with the 16 residue synthetic peptide and to give positive CSP reactions. R32tet$_{32}$ and R32LA are preferred, because of their relative homogeneity, expression levels, and ease of preparation.

Of primary interest in any synthetically produced vaccine, is whether antibody produced against the synthetic immunogen will recognize the authentic molecule and whether the antibody will possess the necessary biological properties to confer protection. The Examples showing both an immunofluorescence assay and the CSP reaction demonstrate that antibody produced against the E. coli constructs reacts with the surface of the sporozoite and thus recognizes authentic CS protein. The presence of CSP antibody has been shown in animals and man to be an important correlate of protective immunity. The fact that anti-construct antibodies inhibit sporozoite invasion of human hepatoma cells in vitro is significant. Hollingdale et al., cited above, showed that both Mabs against P. falciparum and P. vivax as well as polyclonal serum from humans immune to these malaria species blocked sporozoite invasion. Blocking of sporozoite invasion in vitro is thus considered to be an assay for protective antibody. Thus, the data collectively demonstrates that the vaccine of the invasion can be used to protect humans from infection by P. falciparum sporozoites.

The immune response to these recombinant proteins as assessed by ELISA titer, surface reactivity (as shown by IFA and CSP) and blocking of sporozoite invasion is enhanced by use of either Complete Freunds Adjuvant or Alum. Complete Freunds Adjuvant can not be used in humans since it causes fever, produces granulomas and results in tuberculin hypersensitivity. Alum is currently used as an adjuvant in established vaccines such as diptheria and tetanus toxoid as well as one of the newest vaccines, Heptitis B. It has proven efficacy and a long history of safe use in man.

Example 13. Vaccine Preparation

An illustrative vaccine is prepared as follows. To a buffered, aqueous solution of 3% aluminum hydroxide (10 mM sodium phosphate, 150 mM NaCl, pH 6.8; sterilized by filtration), the polypeptide of the invention in similar buffer is added with stirring to a final concentration of 100 ug/ml of polypeptide and 1.0 mg/ml of aluminum (Al$^{3+}$). The pH is maintained at 6.6. The mixture is left overnight at about 0°C. Thimersol is added to a final concentration of 0.005%. The pH is checked and adjusted, if necessary, to 6.8.

**Claims**

1. A fusion polypeptide, comprising tandem repeat units of a P. falciparum circumsporozoite (CS) protein, and a non-CS protein repeat sequence characterised in that the non-CS protein repeat sequences are selected from any of:

Gly; Leu-Arg, influenza virus nonstructural protein 1 (NS1); 81 N-terminal amino acids of NS1; Asn-Thr-Val-Ser-Ser; 86 N-terminal amino acids of a tetracycline resistance gene product (tet$_{86}$); and 32 N-terminal amino acids of a tetracycline resistance gene product (tet$_{32}$) and further characterised in that the tandem repeat units comprises.

[(Asn-Ala-Asn-Pro)$_{15}$(Asn-Val-Asp-Pro)$_1$]$_n$ wherein n ≥ 1.

2. A fusion polypeptide of Claim 1 having the formula N-Met-Asp-Pro[(Asn-Ala-Asn-Pro)$_{15}$(Asn-Val-Asp-Pro)$_1$]$_n$-tet$_{86}$-C, wherein n ≥ 1.

3. A fusion polypeptide of Claim 1 having the formula N-Met-Asp-Pro[Asn-Ala-Asn-Pro)$_{15}$(Asn-Val-Asp-Pro)$_1$]$_n$-tet$_{32}$-C, wherein n ≥ 1.

**4.** The polypeptide of Claim 3, wherein $tet_{32}$ consists of the amino acid sequence

Leu-Arg-Arg-Thr-His-Arg-Gly-Arg-His-His-Arg-Arg-His-Arg-Cys-Gly-Cys-Trp-Arg-Leu-Tyr-Arg-Arg-His-His-Arg-Trp-Gly-Arg-Ser-Gly-Ser-C.

**5.** A fusion polypeptide of Claim 1 having the formula N-Met-Asp-Pro[(Asn-Ala-Asn-Pro)$_{15}$-(Asn-Val-Asp-Pro)$_1$]$_n$Gly-C, wherein $n \geq 1$.

**6.** A fusion polypeptide of Claim 1 having the formula N-Met-Asp-Pro[(Asn-Ala-Asn-Pro)$_{15}$(Asn-Val-Asp-Pro)$_1$]$_n$-Leu-Arg-C, wherein $n \geq 1$.

**7.** A fusion polypeptide of Claim 1 having the formula N-Met-Asp-Pro[(Asn-Ala-Asn-Pro)$_{15}$(Asn-Val-Asp-Pro)$_1$]$_n$-NS1-C wherein $n \geq 1$.

**8.** A fusion polypeptide of Claim 1 having the formula N-NS1$_{81}$-Asp-Pro[(Asn-Ala-Asn-Pro)$_{15}$(Asn-Val-Asp-Pro)$_1$]$_n$-tet$_{32}$-C, wherein $n \geq 1$.

**9.** 'A fusion polypeptide of Claim 1 having the formula N-Met-Asp-Pro[(Asn-Ala-Asn-Pro)$_{15}$(Asn-Val-Asp-Pro)$_1$]$_n$-Asn-Thr-Val-Ser-Ser-C, wherein $n \geq 1$.

**10.** A vaccine for protecting humans against infection by Plasmodium falciparum sporozoite comprising an immunoprotective amount of polypeptide of any of claims 1 to 9 and a pharmaceutically acceptable carrier.

**11.** A polypeptide as claimed in any one of claims 1 to 9 for use in therapy.

**12.** A polypeptide as claimed in any one claims 1 to 9 for use in the prophylaxis of malaria.

**Revendications**

**1.** Polypeptide de fusion, comprenant des unités répétées en tandem d'une protéine de circumsporozoîte (CS) de P. falciparum et une séquence répétée de protéine non-CS, caractérisé en ce que les séquences répétées de protéine non-CS sont choisies parmi l'une quelconque des suivantes :
Gly ; Leu-Arg, protéine 1 non-structurale du virus de l'influenza (NS1) ; 81 acides aminés N-terminaux de NS1 ; Asn-Thr-Val-Ser-Ser ; 86 acides aminés N-terminaux d'un produit du gène de la résistance à la tétracycline (tet$_{86}$) ; et 32 acides aminés N-terminaux d'un produit du gène de la résistance à la tétracycline (tet$_{32}$) et caractérisé de plus en ce que les unités répétées en tandem comprennent : [-(Asn-Ala-Asn-Pro)$_{15}$(Asn-Val-Asp-Pro)$_1$]$_n$ où $n \geq 1$.

**2.** Polypeptide de fusion suivant la revendication 1, ayant la formule N-Met-Asp-Pro[(Asn-Ala-Asn-Pro)$_{15}$-(Asn-Val-Asp-Pro)$_1$]$_n$-tet$_{86}$-C, où $n \geq 1$.

**3.** Polypeptide de fusion suivant la revendication 1, ayant la formule N-Met-Asp-Pro[(Asn-Ala-Asn-Pro)$_{15}$-(Asn-Val-Asp-Pro-)$_1$]$_n$-tet$_{32}$-C, où $n \geq 1$.

**4.** Polypeptide suivant la revendication 3, dans laquelle tet$_{32}$ consiste en la séquence d'acide aminé Leu-Arg-Arg-Thr-His-Arg-Gly-Arg-His-His-Arg-Arg-His-Arg-Cys-Gly-Cys-Trp-Arg-Leu-Tyr-Arg-Arg-His-His-Arg-Trp-Gly-Arg-Ser-Gly-Ser-C.

**5.** Polypeptide de fusion suivant la revendication 1, ayant la formule N-Met-Asp-Pro[(Asn-Ala-Asn-Pro)$_{15}$-(Asn-Val-Asp-Pro)$_1$]$_n$-Gly-C, où $n \geq 1$.

**6.** Polypeptide de fusion suivant la revendication 1, ayant la formule N-Met-Asp-Pro[(Asn-Ala-Asn-Pro)$_{15}$-(Asn-Val-Asp-Pro)$_1$]$_n$-Leu-Arg-C, où $n \geq 1$.

**7.** Polypeptide de fusion suivant la revendication 1, ayant la formule N-Met-Asp-Pro[(Asn-Ala-Asn-Pro)$_{15}$-(Asn-Val-Asp-Pro)$_1$]$_n$-NS1-C, où $n \geq 1$.

**8.** Polypeptide de fusion suivant la revendication 1, ayant la formule N-NS1$_{81}$-Asp-Pro[(Asn-Ala-Asn-Pro)-$_{15}$(Asn-Val-Asp-Pro)$_1$]$_n$-tet$_{32}$-C, où n ≥ 1.

**9.** Polypeptide de fusion suivant la revendication 1, ayant la formule N-Met-Asp-Pro[(Asn-Ala-Asn-Pro)$_{15}$-(Asn-Val-Asp-Pro)$_1$]$_n$-Asn-Thr-Val-Ser-Ser-C, où n ≥ 1.

**10.** Vaccin pour protéger des hommes contre une infection par sporozoïte Plasmodium falciparum, comprenant une quantité immunoprotectrice d'un polypeptide suivant l'une quelconque des revendications 1 à 9 et un support acceptable du point de vue pharmaceutique.

**11.** Polypeptide suivant l'une quelconque des revendications 1 à 9, utile en thérapie.

**12.** Polypeptide suivant l'une quelconque des revendications 1 à 9, utile dans la prophylaxie du paludisme.

**Patentansprüche**

**1.** Fusionspolypeptid, unfassend Tandem-Repeat-Einheiten eines P. falciparum-Circumsporozoiten-(CS)-Proteins und eine Nicht-CS-Protein-Repeat-Sequenz. dadurch **gekennzeichnet**, daß die Nicht-CS-Protein-Repeatsequenzen aus:

Gly; Leu-Arg, Nicht-Strukturprotein 1 (NS1) des Influenza-Virus; 81 N-terminalen Aminosäuren von NS1; Asn-Thr-Val-Ser-Ser; 86 N-terminalen Aminosäuren eines Tetracyclinresistenz-Genprodukts (tet$_{86}$); und 32 N-terminalen Aminosäuren eines Tetracyclinresistenz-Genprodukts (tet$_{32}$), ausgewählt sind,

und weiter dadurch gekennzeichnet, daß die Tandem-Repeat-Einheiten [(Asn-Ala-Asn-Pro)$_{15}$(Asn-Val-Asp-Pro)$_1$]$_n$ umfassen, worin n ≥ 1 ist.

**2.** Fusionspolypeptid nach Anspruch 1 mit der Formel N-Met-Asp-Pro[(Asn-Ala-Asn-Pro)$_{15}$(Asn-Val-Asn-Pro)$_1$]$_n$-tet$_{86}$-C, worin n ≥ 1 ist.

**3.** Fusionspolypeptid nach Anspruch 1 mit der Formel N-Met-Asp-Pro[(Asn-Ala-Asn-Pro)$_{15}$(Asn-Val-Asp-Pro)$_1$]$_n$-tet$_{32}$-C, worin n ≥ 1 ist.

**4.** Polypeptid nach Anspruch 3, worin tet$_{32}$ aus der Aminosäuresequenz Leu-Arg-Arg-Thr-His-Arg-Gly-Arg-His-His-Arg-Arg-His-Arg-Cys-Gly-Cys-Trp-Arg-Leu-Tyr-Arg-Arg-His-His-Arg-Trp-Gly-Arg-Ser-Gly-Ser-C besteht.

**5.** Fusionspolypeptid nach Anspruch 1 mit der Formel N-Met-Asp-Pro[(Asn-Ala-Asn-Pro)$_{15}$-(Asn-Val-Asp-Pro)$_1$]$_n$-Gly-C, worin n ≥ 1 ist.

**6.** Fusionspolypeptid nach Anspruch 1 mit der Formel N-Met-Asp-Pro[(Asn-Ala-Asn-Pro)$_{15}$(Asn-Val-Asp-Pro)$_1$]$_n$-Leu-Arg-C, worin n ≥ 1 ist.

**7.** Fusionspolypeptid nach Anspruch 1 mit der Formel N-Met-Asp-Pro[(Asn-Ala-Asn-Pro)$_{15}$-(Asn-Val-Asp-Pro)$_1$]$_n$-NS1-C, worin n ≥ 1 ist.

**8.** Fusionspolypeptid nach Anspruch 1 mit der Formel N-NS1$_{81}$-Asp-Pro[(Asn-Ala-Asn-Pro)$_{15}$(Asn-Val-Asp-Pro)$_1$]$_n$-tet$_{32}$-C, worin n ≥ 1 ist.

**9.** Fusionspolypeptid nach Anspruch 1 mit der Formel N-Met-Asp-Pro[(Asn-Ala-Asn-Pro)$_{15}$(Asn-Val-Asp-Pro)$_1$]$_n$-Asn-Thr-Val-Ser-Ser-C, worin n ≥ 1 ist.

**10.** Impfstoff zum Schutz von Menschen gegen eine Infektion durch Plasmodium falciparum-Sporozoiten, umfassend eine immunschützende Menge eines Polypeptids nach einem der Ansprüche 1 bis 9 und einen pharmazeutisch verträglichen Träger.

**11.** Polypeptid nach einem der Ansprüche 1 bis 9 zur therapeutischen Verwendung.

**12.** Polypeptid nach einem der Ansprüche 1 bis 9 zur Verwendung bei der Malariaprophylaxe.

FIG. Ia

FIG. Ib